# EUROPÄISCHE PATENTSCHRIFT

(11) **EP 0 989 183 B1**
(45) Veröffentlichungstag und Bekanntmachung des Hinweises auf die Patenterteilung: **13.10.2004**
(21) Anmeldenummer: 99115747.0
(22) Anmeldetag: 10.08.1999
(51) Int. Cl.: C11D 3/50, C11D 3/37, A61K 7/46

(54) **Fixierung von Duftstoffen aus Wasch- und Reinigungsmitteln an harten und weichen Oberflächen**
Fixture of perfumed detergent materials to hard and soft surfaces
Fixage de parfums aux surfaces dures et douces

(30) Priorität: 19.08.1998 DE 19837604
(43) Veröffentlichungstag der Anmeldung: 29.03.2000
(73) Patentinhaber: Henkel KGaA, 40589 Düsseldorf (DE)
(72) Erfinder: Meine, Georg, Dr., 40822 Mettmann (DE); Upadek, Horst, Dr., 40883 Ratingen (DE); Poethkow, Jörg, 40589 Düsseldorf (DE)

(56) Entgegenhaltungen:
- EP-A- 0 256 696
- EP-A- 0 834 551
- WO-A-94/19448
- WO-A-98/52527
- US-A- 3 565 831

## Beschreibung

Die vorliegende Erfindung betrifft die Verwendung von schmutzablösevermögendem Polymer zur Fixierung von Duftstoffen an harten und weichen Oberflächen, wie zum Beispiel an Textilien, insbesondere bei deren Wäsche, sowie ein Verfahren zur Fixierung von Duftstoffen an harten und/oder weichen Oberflächen durch den Einsatz von schmutzablösevermögendem Polymer.

Bei der Wäsche von Textilien beziehungsweise der Reinigung harter Oberflächen wie zum Beispiel Badezimmerfliesen erwartet man nicht nur eine optisch einwandfreie Sauberkeit, sondern auch das Fehlen von etwaigen unangenehmen Gerüchen auf der gereinigten textilen beziehungsweise harten Oberfläche. Im Gegenteil wird oft ein Zurückbleiben von Duftstoffen, die aus dem Reinigungs-, Wasch- oder Wäschenachbehandlungsmittel stammen, als angenehm empfunden und verstärkt den Sauberkeitseindruck. Zum Beispiel beim manuellen Waschen von Textilien, das normalerweise im Waschbecken durchgeführt wird, wird von vielen Anwendern der zurückbleibende Geruch im Becken sowie an den Händen als angenehm empfunden.

Aus der internationalen Patentanmeldung WO 95/04809 ist ein Verfahren zum Parfümieren von Textilien beim Waschen mit lipasehaltigen Waschmitteln bekannt, wobei bestimmte estergruppenhaltige Duftstoffe eingesetzt werden. Die europäische Patentanmeldung EP 0 430 315 betrifft lipasehaltige Waschmittel, die Duftstoffe enthalten, wobei bestimmte Duftstoffkomponenten eine gewisse Gehaltsobergrenze nicht überschreiten sollen, während andere Duftstoffkomponenten eine gewisse Gehaltsuntergrenze nicht unterschreiten dürfen. Dadurch soll sowohl der Lipase-Eigengeruch als auch der Geruch der lipolytisch aus Fetten entstehenden Produkte überdeckt werden.

An Inhaltsstoffe moderner Wasch- und Reinigungsmittel werden üblicherweise aber höhere Anforderungen gestellt als daß sie nur das subjektive Empfinden des Anwenders beeinflussen sollen. So sollen möglichst alle Bestandteile des Mittels zum Wasch- beziehungsweise Reinigungsergebnis beitragen.

Waschmittel enthalten neben den für den Waschprozess unverzichtbaren Inhaltsstoffen wie Tensiden und Buildermaterialien in der Regel weitere Bestandteile, die man unter dem Begriff Waschhilfsstoffe zusammenfassen kann und die so unterschiedliche Wirkstoffgruppen wie Schaumregulatoren, Vergrauungsinhibitoren, Bleichmittel, Bleichaktivatoren und Farbübertragungsinhibitoren umfassen. Zu derartigen Hilfsstoffen gehören auch Substanzen, welche der Wäschefaser schmutzabstoßende Eigenschaften verleihen und die, falls während des Waschvorgangs anwesend, das Schmutzablösevermögen der übrigen Waschmittelbestandteile unterstützen. Gleiches gilt sinngemäß auch für Reinigungsmittel für harte Oberflächen. Derartige schmutzablösevermögende Substanzen werden oft als "Soil-Release"-Wirkstoffe oder wegen ihres Vermögens, die behandelte Oberfläche, zum Beispiel der Faser, schmutzabstoßend auszurüsten, als "Soil-Repellents" bezeichnet. Wegen ihrer chemischen Ähnlichkeit zu Polyesterfasern besonders wirksame schmutzablösevermögende Wirkstoffe, die aber auch bei Geweben beziehungsweise Oberflächen aus anderem Material die erwünschte Wirkung zeigen können, sind Copolyester, die Dicarbonsäureeinheiten, Alkylenglykoleinheiten und Polyalkylenglykoleinheiten enthalten. Schmutzablösevermögende Copolyester der genannten Art wie auch ihr Einsatz in Waschmitteln sind seit langer Zeit bekannt.

Die internationale Patentanmeldung WO 94/19448 beansprucht Waschmitteladditive, die Duftstoffe und Polymere enthalten. Die Polymere wirken schmutzablösend und fixieren die Duftstoffe. Eine Verwendung der Duftstoffe zur Erhöhung der Waschleistung wird nicht erwähnt.

Das US Patent US 3,565,831 beschreibt die Fixierung von Tannenzapfenduft an künstlichen Weihnachtsbäumen mit Hilfe von Celluloseethem. Eien Verwendung von Celluloseethern im Zusammenhang mit Wasch- bzw. Reinigungsvorgängen wird nicht erwähnt.

Die europäische Patentanmeldung EP 0 834 551 A2 beansprucht die Fixierung von Duftstoffen mit Hilfe von Alkylpolyglykosiden. Die Verwendung von bestimmten (Co-)Polyestern wird in dieser Schrift nicht behandelt.

Die internationale Patentanmedlung WO 98/52527 beschreibt die Fixierung von Duftstoffen mit Hilfe der Polymere Polyvinylpyrrolidon und Hydroxypropylcellulose. Eine Anwendung in Waschmitteln wird nicht beschrieben.

So beschreibt zum Beispiel die deutsche Offenlegungsschrift DT 16 17 141 ein Waschverfahren unter Einsatz von Polyethylenterephthalat-Polyoxyethylenglykol-Copolymeren. Die deutsche Offenlegungsschrift DT 22 00 911 betrifft Waschmittel, die Niotensid und ein Mischpolymer aus Polyoxyethylenglykol und Polyethylenterephthalat enthalten. In der deutschen fenlegungsschrift DT 22 53 063 sind saure Textilausrüstungsmittel genannt, die ein Copolymer aus einer dibasigen Carbonsäure und einem Alkylen- oder Cycloalkylenpolyglykol sowie gegebenenfalls einem Alkylen- oder Cycloalkylenglykol enthalten. Polymere aus Ethylenterephthalat und Polyethylenoxid-terephthalat, in denen die Polyethylenglykol-Einheiten Molgewichte von 750 bis 5000 aufweisen und das Molverhältnis von Ethylenterephthalat zu Polyethylenoxid-terephthalat

50:50 bis 90:10 beträgt, und deren Einsatz in Waschmitteln ist in der deutschen Patentschrift DE 28 57 292 beschrieben. Polymere mit Molgewicht 15 000 bis 50 000 aus Ethylenterephthalat und Polyethylenoxid-terephthalat, wobei die Polyethylenglykol-Einheiten Molgewichte von 1000 bis 10 000 aufweisen und das Molverhältnis von Ethylenterephthalat zu Polyethylenoxid-terephthalat 2:1 bis 6:1 beträgt, können gemäß der deutschen Offenlegungsschrift DE 33 24 258 in Waschmitteln eingesetzt werden. Das europäische Patent EP 066 944 betrifft Textilbehandlungsmittel, die einen Copolyester aus Ethylenglykol, Polyethylenglykol, aromatischer Dicarbonsäure und sulfonierter aromatischer Dicarbonsäure in bestimmten Molverhältnissen enthalten. Aus dem europäischen Patent EP 0 185 427 sind Methyl- oder Ethylgruppen-endverschlossene Polyester mit Ethylen-und/oder Propylen-terephthalat- und Polyethylenoxid-terephthalat-Einheiten und Waschmitel, die derartiges Soil-release-Polymer enthalten, bekannt. Das europäische Patent EP 0 241 984 betrifft einen Polyester, der neben Oxyethylen-Gruppen und Terephthalsäureeinheiten auch substituierte Ethyleneinheiten sowie Glycerineinheiten enthält. Aus dem europäischen Patent EP 0 241 985 sind Polyester bekannt, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind. Die europäische Patentschrift EP 0 253 567 betrifft Soil-release-Polymere mit einer Molmasse von 900 bis 9000 aus Ethylenterephthalat und Polyethylenoxid-terephthalat, wobei die Polyethylenglykol-Einheiten Molgewichte von 300 bis 3000 aufweisen und das Molverhältnis von Ethylenterephthalat zu Polyethylenoxid-terephthalat 0,6 bis 0,95 beträgt. Aus der europäischen Patentanmeldung EP 0 272 033 sind zumindest anteilig durch C₁₋₄-Alkyl- oder Acylreste endgruppenverschlossene Polyester mit Poly-propylenterephthalat- und Polyoxyethylenterephthalat-Einheiten bekannt. Das europäische Patent EP 0 274 907 beschreibt sulfoethyl-endgruppenverschlossene terephthalathaltige Soil-release-Polyester. In der europäischen Patentanmeldung EP 0 357 280 werden durch Sulfonierung ungesättigter Endgruppen Soil-Release-Polyester mit Terephthalat-, Alkylenglykol- und Poly-C₂₋₄-Glylkol-Einheiten hergestellt. Die internationale Patentanmeldung WO 95/32232 betrifft schmutzablösevermögende Polyester der allgemeinen Formel

X-(O-(CHR-)ₐ)_{b}[O-OC-Ph-CO-(O-(CHR-)ₒ)ₚ]_{y}O-Y (I)

in der
- a: eine Zahl von 2 bis 8,
- b: eine Zahl von 1 bis 300,
- o: eine Zahl von 2 bis 8,
- p: eine Zahl von 1 bis 300 und
- y: eine Zahl von 1 bis 500 bedeutet,
- Ph: ein o-, m- oder p-Phenylenrest ist, der 1 bis 4 Substituenten, ausgewählt aus Alkylresten mit 1 bis 22 C-Atomen, Sulfonsäuregruppen, Carboxylgruppen und deren Mischungen, tragen kann,
- R: ausgewählt wird aus Wasserstoff, einem Alkylrest mit 1 bis 22 C-Atomen und deren Mischungen, und
- X: und
- Y: unabhängig voneinander aus Wasserstoff, Alkyl- und Arylmonocarbonsäureresten mit 5 bis 32 C-Atomen, Hydroxymonocarbonsäureresten mit 2 bis 22 C-Atomen und einem Oligomerisierungsgrad von 1 bis 100 sowie Dicarbonsäurehalbesterresten, deren zweite Carbonsäuregruppe mit einem Alkohol A-(OCHZCH₂)_{d}-OH verestert ist, bei dem A einen Alkyl- oder Alkenylrest mit 8 bis 22 C-Atomen, Z Wasserstoff oder einen Alkylrest mit 1 bis 2 C-Atomen und d eine Zahl von 1 bis 40 bedeutet, mit der Maßgabe, daß X und Y nicht gleichzeitig Wasserstoff sind, wenn R Wasserstoff oder ein Alkylrest mit 1 C-Atom, a und/oder o 2 und b und/oder p 1 ist, ausgewählt werden. Aus der internationalen Patentanmeldung WO 97/31085 sind soil-repellent-Wirkstoffe für Materialien aus Baumwolle bekannt, die mehrere funktionelle Einheiten aufweisen müssen: Eine erste Einheit, die beispielsweise kationisch sein kann, ist zur Adsorption auf die Baumwolloberfläche durch elektrostatische Wechselwirkung befähigt, und eine zweite Einheit, die hydrophob ausgebildet ist, ist verantwortlich für das Verbleiben des Wirkstoffs an der Wasser/Baumwolle-Grenzfläche. Obwohl es sich bei den dort offenbarten Wirkstoffen nicht ausschließlich um Polymere im Sinne der chemischen Definition dieses Begriffs handelt, sollen sie im Rahmen der vorliegenden Anmeldung wegen ihrer entsprechenden Funktion ebenfalls zu den schmutzablösevermögenden Polymeren gerechnet werden.

Überraschenderweise wurde nun gefunden, daß durch den Einsatz von schmutzablösevermögenden Polymeren die Haftung von Duftstoffen an Oberflächen, zum Beispiel von Textilien, harten Gegenständen oder des menschlichen Körpers, verbessert werden kann, wenn man sie zusammen mit derartigen Duftstoffen beim Waschen beziehungsweise Reinigen verwendet. Umgekehrt wird überraschenderweise die Leistung der schmutzablösevermögenden Polymere, insbesondere bei niedrigen Temperaturen, durch den Einsatz der Duftstoffe erhöht.

Gegenstand der Erfindung ist demnach die Verwendung von oben definierten schmutzablösevermögenden Polymeren zur Fixierung von Duftstoffen an harten und/oder weichen Oberflächen beim Wasch bzw. Reinigen sowie bei der Körperpflege, wobei es sich bei den schmutzablösevermögenden Polymeren um Polyester und/oder Copolyester handelt, welche ausgewählt sind aus der Gruppe umfassend
a) Copolyester, die Dicarbonsäureeinheiten, Alkylenglykoleinheiten und Polyalkylenglykoleinheiten enthalten
b) Polyethylenterephthalat-Polyoxyethylenglykol-Copolymeren
c) Copolymer aus einer dibasigen Carbonsäure und einem Alkylen- oder Cycloalkylenpolyglykol sowie gegebenenfalls einem Alkylen- oder Cycloalkylenglykol
d) Copolyester aus Ethylenglykol, Polyethylenglykol, aromatischer Dicarbonsäure und sulfonierter aromatischer Dicarbonsäure
e) Methyl- oder Ethylgruppen-endverschlossene Polyester mit Ethylen-und/oder Propylen-terephthalat- und Polyethylenoxid-terephthalat-Einheiten
f) Polyester, der neben Oxyethylen-Gruppen und Terephthalsäureeinheiten auch substituierte Ethyleneinheiten sowie Glycerineinheiten enthält
g) Polyester, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind
h) zumindest anteilig durch C₁-C₄-Alkyl- oder Acylreste endgruppenverschlossene Polyester mit Poly-propylenterephthalat- und Polyoxyethylenterephthalat-Einheiten
i) sulfoethyl-endgruppenverschlossene terephthalathaltige Polyester
j) Polyester, hergestellt durch Sulfonierung ungesättigter Endgruppen mit Terephthalat-, Alkylenglykol- und Poly-C₂-C₄-Glylkol-Einheiten, und/oder
k) Polyester der allgemeinen Formel (I)

   X-(O-(CHR-)ₐ)_{b}[O-OC-Ph-CO-(O-(CHR-)ₒ)ₚ]_{y}O-Y (I)
in der a eine Zahl von 2 bis 8, b eine Zahl von 1 bis 300, o eine Zahl von 2 bis 8, p eine Zahl von 1 bis 300 und y eine Zahl von 1 bis 500 bedeutet, Ph ein o-, m- oder p-Phenylenrest ist, der 1 bis 4 Substituenten, ausgewählt aus Alkylresten mit 1 bis 22 C-Atomen, Sulfonsäuregruppen, Carboxylgruppen und deren Mischungen, tragen kann, R ausgewählt wird aus Wasserstoff, einem Alkylrest mit 1 bis 22 C-Atomen und deren Mischungen, und X und Y unabhängig voneinander aus Wasserstoff, Alkyl- und Arylmonocarbonsäureresten mit 5 bis 32 C-Atomen, Hydroxymonocarbonsäureresten mit 2 bis 22 C-Atomen und einem Oligomerisierungsgrad von 1 bis 100 sowie Dicarbonsäurehalbesterresten, deren zweite Carbonsäuregruppe mit einem Alkohol A-(OCHZCH₂)_{d}-OH verestert ist, bei dem A einen Alkyl- oder Alkenylrest mit 8 bis 22 C-Atomen, Z Wasserstoff oder einen Alkylrest mit 1 bis 2 C-Atomen und d eine Zahl von 1 bis 40 bedeutet, mit der Massgabe, dass X und Y nicht gleichzeitig Wasserstoff sind, wenn R Wasserstoff oder ein Alkylrest mit 1 C-Atom, a und/oder o 2 und b und/oder p 1 ist, ausgewählt werden.

Unter weichen Oberflächen sollen in diesem Zusammenhang sowohl menschliche Haut als auch Haar und Textilien unterschiedlicher Zusammensetzung, zum Beispiel aus Baumwolle, Wolle, Seide, Polyester und Mischgeweben jeglicher Art, verstanden werden. Zu harten O-beflächen gehören beispielsweise Badezimmerfliesen, Kacheln oder sonstige keramische Gegenstände in sanitären Einrichtungen. Vorzugsweise wird das schmutzablösevermögende Polymer in Gewichtsmengen, bezogen auf den Duftstoff, im Bereich von 1:10 bis 10:1, insbesondere von 1:1 bis 1:5, verwendet.

Die Verwendung von Duftstoffen zur Erhöhung der Wasch- und/oder Reinigungsleistung von schmutzablösevermögenden Polymeren der oben beschriebenen Art stellt eine bevorzugte Ausführungform der Erfindung dar.

Als Parfümöle in Frage kommen natürliche und synthetische Riechstoffe und deren Gemische. Zu den natürlichen Riechstoffen gehören Extrakte von Blüten (zum Beispiel Lilie, Lavendel, Rosen, Jasmin, Neroli, Ylang-Ylang), Stengeln und Blättern (zum Beispiel Geranium, Patchouli, Petitgrain), Früchten (zum Beispiel Anis, Koriander, Kümmel, Wacholder), Fruchtschalen (zum Beispiel Bergamotte, Zitrone, Orangen), Wurzeln (zum Beispiel Macis, Angelica, Sellerie, Kardamon, Costus, Iris, Calmus), Hölzern (zum Beispiel Pinien-, Sandel-, Guajak-, Zedern-, Rosenholz), Kräutern und Gräsern (zum Beispiel Estragon, Lemongras, Salbei, Thymian), Nadeln und Zweigen (zum Beispiel Fichte, Tanne, Kiefer, Latschen), Harzen und Balsamen (zum Beispiel Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind zum Beispiel Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden zum Beispiel die linearen Alkanale mit 8 - 18 C-Atomen, Citral, Citronellal, Citronellyloxyacetaldehyd, Cyclamenaldehyd, Hydroxycitronellal, Lilial und Bourgeonal, zu den Ketonen zum Beispiel die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Auch ätherische Öle geringerer Flüchtigkeit, die meist als Aromakomponenten verwendet werden, eignen sich als Parfümöle, zum Beispiel Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl.

Zu den vorzugsweise eingesetzten Riechstoffen gehören Bergamotteöl, Dihydromyrcenol. Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat, allein oder in Mischungen untereinander.

Derartige Riechstoffe kommen zur erfindungsgemäßen Verwendung vorzugsweise als Bestandteil von Wasch-, Reinigungs- oder Körperpflegemitteln zum Einsatz. Ein weiterer Gegenstand der Erfindung ist ein Verfahren zur Fixierung von Duftstoffen an harten und/oder weichen Oberflächen, insbesondere an Textilien, welches dadurch gekennzeichnet ist, daß man die Oberfläche mit dem Duftstoff und einem schmutzablösevermögenden Polymer, insbesondere unter Einwirkung von mechanischen Kräften wie sie beispielsweise in haushaltsüblichen Waschmaschinen auftreten, in Gegenwart von Wasser über einen Zeitraum von 2 Minuten bis 90 Minuten bei einer Temperatur unterhalb von 95 °C, insbesondere im Bereich von 20 °C bis 60 °C behandelt, mit der Maßgabe, daß es sich bei dem schmutzablösevermögenden Polymer um Polyester und/oder Copolyester handelt, welches ausgewählt ist aus der Gruppe umfassend
a) Copolyester, die Dicarbonsäureeinheiten, Alkylenglykoleinheiten und Polyalkylenglykoleinheiten enthalten
b) Polyethylenterephthalat-Polyoxyethylenglykol-Copolymeren
c) Copolymer aus einer dibasigen Carbonsäure und einem Alkylen- oder Cycloalkylenpolyglykol sowie gegebenenfalls einem Alkylen- oder Cycloalkylenglykol
d) Copolyester aus Ethylenglykol, Polyethylenglykol, aromatischer Dicarbonsäure und sulfonierter aromatischer Dicarbonsäure
e) Methyl- oder Ethylgruppen-endverschlossene Polyester mit Ethylen-und/oder Propylen-terephthalat- und Polyethylenoxid-terephthalat-Einheiten
f) Polyester, der neben Oxyethylen-Gruppen und Terephthalsäureeinheiten auch substituierte Ethyleneinheiten sowie Glycerineinheiten enthält
g) Polyester, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind
h) zumindest anteilig durch C₁-C₄-Alkyl- oder Acylreste endgruppenverschlossene Polyester mit Poly-propylenterephthalat- und Polyoxyethylenterephthalat-Einheiten
i) sulfoethyl-endgruppenverschlossene terephthalathaltige Polyester
j) Polyester, hergestellt durch Sulfonierung ungesättigter Endgruppen mit Terephthalat-, Alkylenglykol- und Poly-C₂-C₄-Glylkol-Einheiten, und/oder
k) Polyester der allgemeinen Formel (I)

   X-(O-(CHR-)ₐ)_{b}[O-OC-Ph-CO-(O-(CHR-)ₒ)ₚ]_{y}O-Y (I)

   in der a eine Zahl von 2 bis 8, b eine Zahl von 1 bis 300, o eine Zahl von 2 bis 8, p eine Zahl von 1 bis 300 und y eine Zahl von 1 bis 500 bedeutet, Ph ein o-, m- oder p-Phenylen-rest ist, der 1 bis 4 Substituenten, ausgewählt aus Alkylresten mit 1 bis 22 C-Atomen, Sulfonsäuregruppen, Carboxylgruppen und deren Mischungen, tragen kann, R ausgewählt wird aus Wasserstoff, einem Alkylrest mit 1 bis 22 C-Atomen und deren Mischungen, und X und Y unabhängig voneinander aus Wasserstoff, Alkyl- und Arylmonocarbonsäureresten mit 5 bis 32 C-Atomen, Hydroxymonocarbonsäureresten mit 2 bis 22 C-Atomen und einem Oligomerisierungsgrad von 1 bis 100 sowie Dicarbonsäurehalbesterresten, deren zweite Carbonsäuregruppe mit einem Alkohol A-(OCHZCH₂)_{d}-OH verestert ist, bei dem A einen Alkyl- oder Alkenylrest mit 8 bis 22 C-Atomen, Z Wasserstoff oder einen Alkylrest mit 1 bis 2 C-Atomen und d eine Zahl von 1 bis 40 bedeutet, mit der Massgabe, dass X und Y nicht gleichzeitig Wasserstoff sind, wenn R Wasserstoff oder ein Alkylrest mit 1 C-Atom, a und/oder o 2 und b und/oder p 1 ist, ausgewählt werden.

Bevorzugt sind Behandlungsdauern im Bereich von 5 Minuten bis 60 Minuten, insbesondere von 15 Minuten bis 45 Minuten. Die Temperatur liegt vorzugsweise im Bereich von 20 °C bis 60 °C, insbesondere von 20 °C bis 40 °C, wobei besonders bevorzugt während der gesamten Behandlungszeit der Oberfläche mit der erfindungsgemäß verwendeten Kombination die Temperatur im Bereich von 20 °C bis 40 °C liegt. Die Konzentration des schmutzablösevermögenden Polymers in der wäßrigen Behandlungsflotte liegt beim erfindungsgemäßen Verfahren vorzugsweise im Bereich von 0,01 g/l bis 0,25 g/l, insbesondere von 0,05 g/l bis 0,15 g/l, während die Konzentration an Duftstoff in der wäßrigen Behandlungsflotte vorzugsweise im Bereich von 0,004 g/l bis 0,12 g/l, insbesondere von 0,02 g/l bis 0,04 g/l liegt.

Besonders vorteilhaft kann das erfindungsgemäße Verfahren mit Hilfe eines Wasch- beziehungsweise Reinigungsmittels durchgeführt werden, welches einen oben definierten geeigneten Duftstoff und das schmutzablösevermögende Polymer enthält. Alternativ kann das erfindungsgemäße Verfahren auch durch Anwendung mindestens zweier gegebenenfalls übliche sonstige Wasch- beziehungsweise Reinigungsmittelinhaltsstoffe enthaltender Mittel, deren eines den Duftstoff und deren zweites das schmutzablösevermögende Polymer enthält, ausgeführt werden. Derartige Mittel können als solche oder nach Verdünnen mit Wasser im erfindungsgemäßen Verfahren beziehungsweise im Rahmen der erfindungsgemäßen Verwendung eingesetzt werden. In einer bevorzugten Ausgestaltung der Erfindung kommt der Duftstoff in Gestalt von in der deutschen Patentanmeldung DE 197 46 780 beschriebenen Duft stoff-Formkörpern, insbesondere Duftperlen, mit Schüttgewichten oberhalb von 700 g/l zum Einsatz, die wie dort beschrieben durch Granulation oder Preßagglomeration eines festen und im wesentlichen wasserfreien Vorgemischs aus 65 bis 95 Gew.-% Trägerstoff(en), 0 bis 10 Gew.-% Hilfsstoff(en) sowie 5 bis 25 Gew.-% Parfüm hergestellt werden können.

Bei einem Mittel für den Einsatz im erfindungsgemäßen Verfahren kann es sich insbesondere um ein Textilwaschmittel, welches in teilchenförmiger oder flüssiger Form vorliegen kann, ein in entsprechender Form vorliegendes Reinigungsmittel für harte Oberflächen, zum Beispiel einen Bad- oder Sanitärreiniger, oder ein Reinigungsmittel für den menschlichen Körper, zum Beispiel ein Haarshampoo, eine Reinigungslotion oder eine Stückseife, handeln. Insbesondere ist die erfindungsgemäße Lehre im Körperpflegebereich aber einsetzbar für Dauerwellpräparate und Depilatorien sowie für Mittel, die, zum Beispiel zur Einstellung des pH-Wertes, niedere Amine enthalten.

Im Rahmen der Erfindung geeignete schmutzablösevermögende Polymere und ihre Herstellung werden zum Beispiel in den eingangs zitierten Druckschriften beschrieben. Zu den bevorzugten schmutzablösevermögenden Polymeren im Sinne der vorliegenden Erfindung gehört das von der Firma Rhone-Poulenc vertriebene nichtionische Polymer TCG-1 auf Basis eies aminofunktionalen Silikons. Insbesondere bei Einsatz dieses Polymers in der Textilwäsche beobachtet man bei Fehlen üblicher für diesen Effekt brauchbarer Wirkstoffe, zum Beispiel aus der Gruppe der Kationtenside, als Zusatznutzen bereits nach wenigen Wäschen einen angenehm weichen Griff der Wäsche. Schmutzablösevermögende Polymere sind in erfindungsgemäß zu verwendenden Mitteln vorzugsweise in Mengen von 0,2 Gew.-% bis 2 Gew.-%, insbesondere von 0,5 Gew.-% bis 1,5 Gew.-% enthalten. Das Gewichtsverhältnis von schmutzablösevermögendem Polymer zu Duftstoff beträgt in erfindungsgemäßen Mitteln vorzugsweise 10:1 bis 1:2, insbesondere 2:1 bis 1:1.

Besonders ausgeprägt zeigt sich die reinigungsverstärkende Wirkung der Duftstoffe bei Verschmutzungen auf Mineralölbasis, die von Lipasen naturgemäß nicht hydrolytisch angegriffen werden können. In einer bevorzugten Ausgestaltung der Erfindung wird der Duftstoff daher in Gegenwart einer Lipase verwendet, so daß sowohl mineralölbasierte als auch glyzerinesterbasierte fetthaltige Anschmutzungen, zum Beispiel an Textilien, gut entfernt werden. Vorzugsweise enthält ein erfindungsgemäßes Mittel Lipase in einer solchen Menge, daß es eine lipolytische Aktivität im Bereich von 2 LU bis 2000 LU, insbesondere von 2 LU bis 100 LU pro Gramm des Mittels aufweist.

Zusätzlich können erfindungsgemäße beziehungsweise im erfindungsgemäßen Verfahren eingesetzte Mittel, die als insbesondere pulverförmige Feststoffe, in nachverdichteter Teilchenform, als homogene Lösungen oder Suspensionen vorliegen können, alle üblichen Inhaltsstoffe aufweisen, solange sie nicht mit dem schmutzablösevermögenden Polymer oder insbesondere dem Dufststoff so in Wechselwirkung treten, daß der erwünschte Effekt der Verstärkung der Duftfixierung an der Oberfläche ausbleibt.

Erfindungsgemäß zu verwendende Wasch- oder Reinigungsmittel können insbesondere Buildersubstanzen, oberflächenaktive Tenside, Enzyme, organische und/oder anorganische Persauerstoffverbindungen, Persauerstoff-Aktivatoren, wassermischbare organische Lösungsmittel, Sequestrierungsmittel, Elektrolyte, pH-Regulatoren und weitere Hilfsstoffe, wie optische Aufheller, Vergrauungsinhibitoren, Farbübertragungsinhibitoren, Schaumregulatoren sowie Farbstoffe enthalten.

Die Mittel können Tenside enthalten, wobei insbesondere anionische Tenside, nichtionische Tenside und deren Gemische in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder Sulfonat-Gruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Derartige Tenside sind in den erfindungsgemäß zu verwendenden Waschmitteln in Mengenanteilen von vorzugsweise 5 Gew.-% bis 50 Gew.-%, insbesondere von 8 Gew.-% bis 30 Gew.-%, enthalten.

Ein erfindungsgemäß zu verwendendes Mittel, insbesondere wenn es für die Textilwäsche zum Einsatz kommen soll, enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, insbesondere die durch Oxidation von Polysacchariden zugänglichen Polycarboxylate der europäischen Patentschrift EP 0 609 273, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Die relative Molekülmasse der Homopolymeren ungesättiger Carbonsäuren liegt im allgemeinen zwischen 5 000 und 200 000, die der Copolymeren zwischen 2 000 und 200 000, vorzugsweise 50 000 bis 120 000, jeweils bezogen auf freie Säure. Ein besonders bevorzugtes Acrylsäure-Maleinsäure-Copolymer weist eine relative Molekülmasse von 50 000 bis 100 000 auf. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Als wasserlösliche organische Buildersubstanzen können auch Terpolymere eingesetzt werden, die als Monomere zwei ungesättigte Säuren und/oder deren Salze sowie als drittes Monomer Vinylalkohol und/oder ein Vinylalkohol-Derivat oder ein Kohlenhydrat enthalten. Das erste saure Monomer beziehungsweise dessen Salz leitet sich von einer monoethylenisch ungesättigten C₃-C₈-Carbonsäure und vorzugsweise von einer C₃-C₄-Monocarbonsäure, insbesondere von (Meth)-acrylsäure ab. Das zweite saure Monomer beziehungsweise dessen Salz kann ein Derivat einer C₄-C₈-Dicarbonsäure sein, wobei Maleinsäure besonders bevorzugt ist. Die dritte monomere Einheit wird in diesem Fall von Vinylalkohol und/oder vorzugsweise einem veresterten Vinylalkohol gebildet. Insbesondere sind Vinylalkohol-Derivate bevorzugt, welche einen Ester aus kurzkettigen Carbonsäuren, beispielsweise von C₁-C₄-Carbonsäuren, mit Vinylalkohol darstellen. Bevorzugte Polymere enthalten dabei 60 Gew.-% bis 95 Gew.-%, insbesondere 70 Gew.-% bis 90 Gew.-% (Meth)acrylsäure bzw. (Meth)acrylat, besonders bevorzugt Acrylsäure bzw. Acrylat, und Maleinsäure bzw. Maleinat sowie 5 Gew.-% bis 40 Gew.-%, vorzugsweise 10 Gew.-% bis 30 Gew.-% Vinylalkohol und/oder Vinylacetat. Ganz besonders bevorzugt sind dabei Polymere, in denen das Gewichtsverhältnis von (Meth)acrylsäure beziehungsweise (Meth)acrylat zu Maleinsäure beziehungsweise Maleinat zwischen 1:1 und 4:1, vorzugsweise zwischen 2:1 und 3:1 und insbesondere 2:1 und 2,5:1 liegt. Dabei sind sowohl die Mengen als auch die Gewichtsverhältnisse auf die Säuren bezogen. Das zweite saure Monomer beziehungsweise dessen Salz kann auch ein Derivat einer Allylsulfonsäure sein, die in 2-Stellung mit einem Alkylrest, vorzugsweise mit einem C₁-C₄-Alkylrest, oder einem aromatischen Rest, der sich vorzugsweise von Benzol oder Benzol-Derivaten ableitet, substituiert ist. Bevorzugte Terpolymere enthalten dabei 40 Gew.-% bis 60 Gew.-%, insbesondere 45 bis 55 Gew.-% (Meth)acrylsäure beziehungsweise (Meth)acrylat, besonders bevorzugt Acrylsäure beziehungsweise Acrylat, 10 Gew.-% bis 30 Gew.-%, vorzugsweise 15 Gew.-% bis 25 Gew.-% Methallylsulfonsäure bzw. Methallylsulfonat und als drittes Monomer 15 Gew.-% bis 40 Gew.-%, vorzugsweise 20 Gew.-% bis 40 Gew.-% eines Kohlenhydrats. Dieses Kohlenhydrat kann dabei beispielsweise ein Mono-, Di-, Oligooder Polysaccharid sein, wobei Mono-, Di- oder Oligosaccharide bevorzugt sind. Besonders bevorzugt ist Saccharose. Durch den Einsatz des dritten Monomers werden vermutlich Sollbruchstellen in das Polymer eingebaut, die für die gute biologische Abbaubarkeit des Polymers verantwortlich sind. Diese Terpolymere lassen sich insbesondere nach Verfahren herstellen, die in den deutschen Patentschriften DE 42 21 381 und DE 43 00 772 beschrieben sind, und weisen im allgemeinen eine relative Molekülmasse zwischen 1 000 und 200 000, vorzugsweise zwischen 200 und 50 000 und insbesondere zwischen 3 000 und 10 000 auf Weitere bevorzugte Copolymere sind solche, die in den deutschen Patentanmeldungen DE 43 03 320 und DE 44 17 734 beschrieben werden und als Monomere vorzugsweise Acrolein und Acrylsäure/Acrylsäuresalze beziehungsweise Vinylacetat aufweisen. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Derartige organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, Mitteln eingesetzt.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien werden insbesondere kristalline oder amorphe Alkalialumosilikate, in Mengen von bis zu 50 Gew.-%, vorzugsweise nicht über 40 Gew.-% und in flüssigen Mitteln insbesondere von 1 Gew.-% bis 5 Gew.-%, eingesetzt. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das nach den Angaben der deutschen Patentschrift DE 24 12 837 bestimmt werden kann, liegt in der Regel im Bereich von 100 bis 200 mg CaO pro Gramm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Solche mit einem molaren Verhältnis Na₂O:SiO₂ von 1:1,9 bis 1:2,8 können nach dem Verfahren der europäischen Patentanmeldung EP 0 425 427 hergestellt werden. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Kristalline Schichtsilikate, die unter diese allgemeine Formel fallen, werden beispielsweise in der europäischen Patentanmeldung EP 0 164 514 beschrieben. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅ · y H₂O) bevorzugt, wobei β-Natriumdisilikat beispielsweise nach dem Verfahren erhalten werden kann, das in der internationalen Patentanmeldung WO 91/08171 beschrieben ist. δ-Natriumsilikate mit einem Modul zwischen 1,9 und 3,2 können gemäß den japanischen Patentanmeldungen JP 04/238 809 oder JP 04/260 610 hergestellt werden. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, herstellbar wie in den europäischen Patentanmeldungen EP 0 548 599, EP 0 502 325 und EP 0 425 428 beschrieben, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es nach dem Verfahren der europäischen Patentanmeldung EP 0 436 835 aus Sand und Soda hergestellt werden kann. Kristalline Natriumsilikate mit einem Modul im Bereich von 1,9 bis 3,5, wie sie nach den Verfahren der europäischen Patentschriften EP 0 164 552 und/oder EP 0 294 753 erhältlich sind, werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind in den erfindungsgemäßen Waschmitteln vorzugsweise in Mengen bis zu 60 Gew.-%, insbesondere von 5 Gew.-% bis 40 Gew.-%, enthalten.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Perverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, eingesetzt. Falls ein erfindungsgemäßes Waschmittel Persauerstoffverbindungen enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten beziehungsweise Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und die aus den deutschen Patentanmeldungen DE 196 16 693 und DE 196 16 767 bekannten Enolester sowie acetyliertes Sorbitol und Mannitol beziehungsweise deren in der europäischen Patentanmeldung EP 0 525 239 beschriebene Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam, die aus den internationalen Patentanmeldungen WO 94/27970, WO 94/28102, WO 94/28103, WO 95/00626, WO 95/14759 und WO 95/17498 bekannt sind. Die aus der deutschen Patentanmeldung DE 196 16 769 bekannten hydrophil substituierten Acylacetale und die in der deutschen Patentanmeldung DE 196 16 770 sowie der internationalen Patentanmeldung WO 95/14075 beschriebenen Acyllactame werden ebenfalls bevorzugt eingesetzt. Auch die aus der deutschen Patentanmeldung DE 44 43 177 bekannten Kombinationen kon-ventioneller Bleichaktivatoren können eingesetzt werden. Derartige Bleichaktivatoren sind im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten.

Zusätzlich zu den oben aufgeführten konventionellen Bleichaktivatoren oder an deren Stelle können auch die aus den europäischen Patentschriften EP 0 446 982 und EP 0 453 003 bekannten Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere die aus der deutschen Patentanmeldung DE 195 29 905 bekannten Mangan-, Eisen-, Cobalt-, Ruthenium- oder Molybdän-Salenkomplexe und deren aus der deutschen Patentanmeldung DE 196 20 267 bekannte N-Analogverbindungen, die aus der deutschen Patentanmeldung DE 195 36 082 bekannten Mangan-, Eisen-, Cobalt-, Ruthenium- oder Molybdän-Carbonylkomplexe, die in der deutschen Patentanmeldung DE 196 05 688 beschriebenen Mangan-, Eisen-, Cobalt-, Ruthenium-, Molybdän-, Titan-, Vanadium- und Kupfer-Komplexe mit stickstoffhaltigen Tripod-Liganden, die aus der deutschen Patentanmeldung DE 196 20 411 bekannten Cobalt-, Eisen-, Kupfer- und Ruthenium-Amminkomplexe, die in der deutschen Patentanmeldung DE 44 16 438 beschriebenen Mangan-, Kupfer- und Cobalt-Komplexe, die in der europäischen Patentanmeldung EP 0 272 030 beschriebenen Cobalt-Komplexe, die aus der europäischen Patentanmeldung EP 0 693 550 bekannten Mangan-Komplexe, die aus der europäischen Patentschrift EP 0 392 592 bekannten Mangan-, Eisen-, Cobalt- und Kupfer-Komplexe und/oder die in der europäischen Patentschrift EP 0 443 651 oder den europäischen Patentanmeldungen EP 0 458 397, EP 0 458 398, EP 0 549 271, EP 0 549 272, EP 0 544 490 und EP 0 544 519 beschriebenen Mangan-Komplexe. Kombinationen aus Bleichaktivatoren und Übergangsmetall-Bleichkatalysatoren sind beispielsweise aus der deutschen Patentanmeldung DE 196 13 103 und der internationalen Patentanmeldung WO 95/27775 bekannt. Bleichverstärkende Übergangsmetallkomplexe, insbesondere mit den Zentralatomen Mn, Fe, Co, Cu, Mo, V, Ti und/oder Ru, werden in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% bis 0,1 Gew.-%, jeweils bezogen auf gesamtes Mittel, eingesetzt.

Als in den Mitteln verwendbare Enzyme kommen anstatt oder neben der oben erwähnten Lipase solche aus der Klasse der Proteasen, Cutinasen, Amylasen, Pullulanasen, Hemicellulasen, Cellulasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können, wie zum Beispiel in den internationalen Patentanmeldungen WO 92/11347 oder WO 94/23005 beschrieben, an Trägerstoffen adsorbiert und/oder in Hüllsubstanzen eingebettet sein, um sie gegen vorzeitige Inaktivierung zu schützen. Sie sind in den erfindungsgemäßen Waschmitteln vorzugsweise nicht über 5 Gew.-%, insbesondere von 0,2 Gew.-% bis 2 Gew.-%, enthalten.

Die Mittel können als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'disulfonsäure oder gleichartig aufgebaute Verbindungen, die anstelle der MorpholinoGruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, zum Beispiel die Alkalisalze des 4,4'-Bis(2-sulfostyryl)-diphenyls, 4,4'-Bis(4-chlor-3-sulfostyryl)-diphenyls oder 4-(4-Chlorstyryl)-4'-(2-sulfostyryl)-diphenyls. Auch Gemische der vorgenannten Aufheller können verwendet werden.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäurealkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffinhaltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Waschmitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammoniumoder Alkalihydroxide, enthalten. Derartige pH-Regulatoren sind in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel bereitet keine Schwierigkeiten und kann in im Prinzip bekannter Weise, zum Beispiel durch Sprühtrocknen oder Granulation, erfolgen, wobei Persauerstoffverbindung und Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist das aus der europäischen Patentschrift EP 0 486 592 bekannte, einen Extrusionsschritt aufweisende Verfahren bevorzugt.

Bei erfindungsgemäß zu verwendenden Körperpflegemitteln handelt es sich in einer ersten bevorzugten Ausführungsform um ein Dauerwellmittel, insbesondere um eine Wellotion. Unter einer Wellotion wird dabei die Zubereitung verstanden, die das Reduktionsmittel enthält. Als Reduktionsmittel werden üblicherweise Mercaptoverbindungen und/oder Salze der schwefligen Säure eingesetzt. Bevorzugte Mercaptoverbindungen sind Thioglykolsäure, ihre physiologisch verträglichen Salze sowie ihre Ester. Weiterhin bevorzugt verwendet werden, wenn auch in geringerem Umfang, Cysteamin, Cystein, Thiomilchsäure, Thioäpfelsäure, Bunte Salze und α-Mercaptoethansulfonsäure.

Zur Überdeckung des Eigengeruchs dieser Mercaptoverbindungen enthalten die Wellotionen üblicherweise Parfümöle. Es besteht jedoch das Problem, daß Reste der Mercaptoverbindungen auch nach dem Fixieren der Dauerwelle am Haar verbleiben, in vielen Fällen sogar deutlich länger als die eingesetzten Parfümöle. Werden diese Haare später mit einem nur gering parfümierten Mittel, zum Beispiel einem Shampoo im Rahmen der üblichen Reinigung, behandelt, so lösen sich diese Reste der Mercaptoverbindung unter entsprechender Duftentfaltung sukzessive vom Haar. Diesem Problem kann durch erfindungsgemäße Verwendung der schmutzablösevermögenden Polymere in den Wellotionen in hervorragender Weise vorgebeugt werden. Die Wellotionen können des weiteren alle dem Fachmann bekannten Inhaltsstoffe, wie beispielsweise anionische Tenside, zwitterionische Tenside, ampholytische Tenside, nichtonische Tenside, kationische Tenside, Proteinhydrolysate, Verdickungsmittel, Strukturanten, kationische, anionische, zwitterionische, amphotere und nichtionische Polymere, Lösungsvermittler, Substanzen zur Einstellung des pH-Wertes, Wirkstoffe wie Allantoin, Pyrrolidoncarbonsäuren und Pflanzenextrakte, Lichtschutzmittel, Komplexbildner, Quell- und Penetrationsstoffe, Trübungsmittel, Farbstoffe, Parfümöle, Perlglanzmittel und Treibmittel. Diese Ausführungen bezüglich Dauerwellmitteln gelten in gleicher Weise auch für Depilatorien.

Gemäß einer weiteren bevorzugten Ausführungsform werden die schmutzablösevermögenden Polymere erfindungsgemäß in solchen Mitteln eingesetzt, die Amine enthalten. Auch hier erleichtern sie die Überdeckung unerwünschter Duftnuancen, die auf diese Amine zurückgehen. Solche Mittel sind neben den bereits oben genannten Dauerwellmitteln beispielsweise Haarfärbemittel.

Gemäß einer dritten bevorzugten Ausführungsform kann die erfindungsgemäße Lehre dazu eingesetzt werden, den Parfümanteil in Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder überhaupt nicht verwenden können. In diesem Zusammenhang sind vor allem Hautpflegeprodukte und Deodoratien zu nennen.

## Patentansprüche

1. Verwendung von schmutzablösevennögenden Polymeren zur Fixierung von Duftstoffen an harten und/oder weichen Oberflächen beim Waschen bzw. Reinigen sowie bei der Körperpflege, **dadurch gekennzeichnet, daß** es sich bei den schmutzablösevermögenden Polymeren um Polyester und/oder Copolyester handelt, welche ausgewählt sind aus der Gruppe umfassend
a) Copolyester, die Dicarbonsäureeinheiten, Alkylenglykoleinheiten und Polyalkylenglykoleinheiten enthalten
b) Polyethylenterephthalat-Polyoxyethylenglykol-Copolymeren
c) Copolymer aus einer dibasigen Carbonsäure und einem Alkylen- oder Cycloalkylenpolyglykol sowie gegebenenfalls einem Alkylen- oder Cycloalkylenglykol
d) Copolyester aus Ethylenglykol, Polyethylenglykol, aromatischer Dicarbonsäure und sulfonierter aromatischer Dicarbonsäure
e) Methyl- oder Ethylgruppen-endverschlossene Polyester mit Ethylenund/oder Propylen-terephthalat- und Polyethylenoxid-terephthalat-Einheiten
f) Polyester, der neben Oxyethylen-Gruppen und Terephthalsäureeinheiten auch substituierte Ethyleneinheiten sowie Glycerineinheiten enthält
g) Polyester, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind
h) zumindest anteilig durch C₁-C₄-Alkyl- oder Acylreste endgruppenverschlossene Polyester mit Poly-propylenterephthalat- und Polyoxyethylenterephthalat-Einheiten
i) sulfoethyl-endgruppenverschlossene terephthalathaltige Polyester
j) Polyester, hergestellt durch Sulfonierung ungesättigter Endgruppen mit Terephthalat-, Alkylenglykol- und Poly-C₂-C₄-Glylkol-Einheiten, und/oder
k) Polyester der allgemeinen Formel (I)
X-(O-(CHR-)ₐ)_{b}[O-OC-Ph-CO-(O-(CHR-)ₒ)ₚ]_{y}O-Y (I)
in der a eine Zahl von 2 bis 8, b eine Zahl von 1 bis 300, o eine Zahl von 2 bis 8, p eine Zahl von 1 bis 300 und y eine Zahl von 1 bis 500 bedeutet, Ph ein o-, m- oder p-Phenylenrest ist, der 1 bis 4 Substituenten, ausgewählt aus Alkylresten mit 1 bis 22 C-Atomen, Sulfonsäuregruppen, Carboxylgruppen und deren Mischungen, tragen kann, R ausgewählt wird aus Wasserstoff, einem Alkylrest mit 1 bis 22 C-Atomen und deren Mischungen, und X und Y unabhängig voneinander aus Wasserstoff, Alkyl- und Arylmonocarbonsäureresten mit 5 bis 32 C-Atomen, Hydroxymonocarbonsäureresten mit 2 bis 22 C-Atomen und einem Oligomerisierungsgrad von 1 bis 100 sowie Dicarbonsäurehalbesterresten, deren zweite Carbonsäuregruppe mit einem Alkohol A-(OCHZCH₂)_{d}-OH verestert ist, bei dem A einen Alkyl- oder Alkenylrest mit 8 bis 22 C-Atomen, Z Wasserstoff oder einen Alkylrest mit 1 bis 2 C-Atomen und d eine Zahl von 1 bis 40 bedeutet, mit der Massgabe, dass X und Y nicht gleichzeitig Wasserstoff sind, wenn R Wasserstoff oder ein Alkylrest mit 1 C-Atom, a und/oder o 2 und b und/oder p 1 ist, ausgewählt werden.

2. Verwendung nach Anspruch 1 zur Fixierung von Duftstoffen an menschlicher Haut, Haar und/oder Textilien beim Waschen bzw. Reinigen sowie bei der Körperpflege.

3. Verwendung nach Anspruch 1 oder 2, **dadurch gekennzeichnet, daß** durch die Duftstoffe die Wasch- und/oder Reinigungsleistung der schmutzablösevermögenden Polymere erhöht wird.

4. Verwendung nach einem der Ansprüche 1 bis 3, **dadurch gekennzeichnet, daß** man das schmutzablösevermögende Polymer in Gewichtsmengen, bezogen auf den Duftstoff, im Bereich von 1:10 bis 10:1, insbesondere von 1:1 bis 1:5, einsetzt.

5. Verfahren zur Fixierung von Duftstoffen an harten und/oder weichen Oberflächen, **dadurch gekennzeichnet, daß** man die Oberfläche mit dem Duftstoff und einem schmutzablösevermögendem Polymer in Gegenwart von Wasser über einen Zeitraum von 2 Minuten bis 90 Minuten bei einer Temperatur unter 95°C behandelt, mit der Maßgabe, daß es sich bei dem schmutzablösevermögenden Polymer um Polyester und/oder Copolyester handelt, welches ausgewählt ist aus der Gruppe umfassend
a) Copolyester, die Dicarbonsäureeinheiten, Alkylenglykoleinheiten und Polyalkylenglykoleinheiten enthalten
b) Polyethylenterephthalat-Polyoxyethylenglykol-Copolymeren
c) Copolymer aus einer dibasigen Carbonsäure und einem Alkylen- oder Cycloalkylenpolyglykol sowie gegebenenfalls einem Alkylen- oder Cycloalkylenglykol
d) Copolyester aus Ethylenglykol, Polyethylenglykol, aromatischer Dicarbonsäure und sulfonierter aromatischer Dicarbonsäure
e) Methyl- oder Ethylgruppen-endverschlossene Polyester mit Ethylenund/oder Propylen-terephthalat- und Polyethylenoxid-terephthalat-Einheiten
f) Polyester, der neben Oxyethylen-Gruppen und Terephthalsäureeinheiten auch substituierte Ethyleneinheiten sowie Glycerineinheiten enthält
g) Polyester, die neben Oxyethylen-Gruppen und Terephthalsäureeinheiten 1,2-Propylen-, 1,2-Butylen- und/oder 3-Methoxy-1,2-propylengruppen sowie Glycerineinheiten enthalten und mit C₁- bis C₄-Alkylgruppen endgruppenverschlossen sind
h) zumindest anteilig durch C₁-C₄-Alkyl- oder Acylreste endgruppenverschlossene Polyester mit Poly-propylenterephthalat- und Polyoxyethylenterephthalat-Einheiten
i) sulfoethyl-endgruppenverschlossene terephthalathaltige Polyester
j) Polyester, hergestellt durch Sulfonierung ungesättigter Endgruppen mit Terephthalat-, Alkylenglykol- und Poly-C₂-C₄-Glylkol-Einheiten, und/oder
k) Polyester der allgemeinen Formel (I)
X-(O-(CHR-)ₐ)_{b}[O-OC-Ph-CO-(O-(CHR-)ₒ)ₚ]_{y}O-Y (I)
in der a eine Zahl von 2 bis 8, b eine Zahl von 1 bis 300, o eine Zahl von 2 bis 8, p eine Zahl von 1 bis 300 und y eine Zahl von 1 bis 500 bedeutet, Ph ein o-, m- oder p-Phenylen-rest ist, der 1 bis 4 Substituenten, ausgewählt aus Alkylresten mit 1 bis 22 C-Atomen, Sulfonsäuregruppen, Carboxylgruppen und deren Mischungen, tragen kann, R ausgewählt wird aus Wasserstoff, einem Alkylrest mit 1 bis 22 C-Atomen und deren Mischungen, und X und Y unabhängig voneinander aus Wasserstoff, Alkyl- und Arylmonocarbonsäureresten mit 5 bis 32 C-Atomen, Hydroxymonocarbonsäureresten mit 2 bis 22 C-Atomen und einem Oligomerisierungsgrad von 1 bis 100 sowie Dicarbonsäurehalbesterresten, deren zweite Carbonsäuregruppe mit einem Alkohol A-(OCHZCH₂)_{d}-OH verestert ist, bei dem A einen Alkyl- oder Alkenylrest mit 8 bis 22 C-Atomen, Z Wasserstoff oder einen Alkylrest mit 1 bis 2 C-Atomen und d eine Zahl von 1 bis 40 bedeutet, mit der Massgabe, dass X und Y nicht gleichzeitig Wasserstoff sind, wenn R Wasserstoff oder ein Alkylrest mit 1 C-Atom, a und/oder o 2 und b und/oder p 1 ist, ausgewählt werden.

6. Verfahren nach Anspruch 5, **dadurch gekennzeichnet, daß** die Behandlungsdauer im Bereich von 5 Minuten bis 60 Minuten, insbesondere von 15 Minuten bis 45 Minuten liegt.

7. Verfahren nach Anspruch 5 oder 6, **dadurch gekennzeichnet, daß** die Temperatur im Bereich von 20 °C bis 60 °C, insbesondere von 20°C bis 40 °C liegt.

8. Verfahren nach einem der Ansprüche 5 bis 7, **dadurch gekennzeichnet, daß** während der gesamten Behandlungszeit die Temperatur im Bereich von 20 °C bis 40 °C liegt.

9. Verfahren nach einem der Ansprüche 5 bis 8, **dadurch gekennzeichnet, daß** die Konzentration des schmutzablösevermögenden Polymers im Bereich von 0,01 g/l bis 0,25 g/l, insbesondere von 0,05 g/l bis 0,15 g/l liegt.

10. Verfahren nach einem der Ansprüche 5 bis 9, **dadurch gekennzeichnet, daß** die Konzentration an Duftstoff in der wäßrigen Behandlungsflotte im Bereich von 0,004 g/l bis 0,12 g/l, insbesondere von 0,02 g/l bis 0,04 g/l liegt.

## Claims

1. Use of soil release polymers for the fixing of fragrances to hard and/or soft surfaces during washing and/or cleaning, and in bodycare, **characterized in that** the soil release polymers are polyesters and/or copolyesters which are chosen from the group comprising
a) copolyesters which comprise dicarboxylic acid units, alkylene glycol units and polyalkylene glycol units
b) polyethylene terephthalate-polyoxyethylene glycol copolymers
c) copolymers of a dibasic carboxylic acid and an alkylene or cycloalkylene polyglycol, and optionally an alkylene or cycloalkylene glycol
d) copolyesters of ethylene glycol, polyethylene glycol, aromatic dicarboxylic acid and sulphonated aromatic dicarboxylic acid
e) methyl- or ethyl-capped polyesters with ethylene terephthalate and/or propylene terephthalate and polyethylene oxide terephthalate units
f) polyesters which, besides oxyethylene groups and terephthalic acid units, also comprise substituted ethylene units and glycerol units
g) polyesters which, besides oxyethylene groups and terephthalic acid units, comprise 1,2-propylene, 1,2-butylene and/or 3-methoxy-1,2-propylene groups and glycerol units and are terminally capped with C₁- to C₄-alkyl groups
h) at least proportionally C₁-C₄-alkyl- or acyl-terminally capped polyesters with polypropylene terephthalate and polyoxyethylene terephthalate units
i) sulphoethyl-terminally capped terephthalate-containing polyesters
j) polyesters prepared by sulphonation of unsaturated end groups with terephthalate, alkylene glycol and/or poly-C₂-C₄-glycol units, and/or
k) polyesters of the general formula (I)
X-(O-(CHR-)ₐ)_{b}[O-OC-Ph-CO-(O-(CHR-)ₒ)ₚ]_{y}O-Y (I)
in which a is a number from 2 to 8, b is a number from 1 to 300, o is a number from 2 to 8, p is a number from 1 to 300 and y is a number from 1 to 500, Ph is an o-, m- or p-phenylene radical which can carry 1 to 4 substituents chosen from alkyl radicals having 1 to 22 carbon atoms, sulphonic acid groups, carboxyl groups and mixtures thereof, R is chosen from hydrogen, an alkyl radical having 1 to 22 carbon atoms and mixtures thereof, and X and Y, independently of one another, are chosen from hydrogen, alkyl- and arylmonocarboxylic acid radicals having 5 to 32 carbon atoms, hydroxymonocarboxylic acid radicals having 2 to 22 carbon atoms and a degree of oligomerization of from 1 to 100, and dicarboxylic monoester radicals whose second carboxylic acid group is esterified by an alcohol A-(OCHZCH₂)_{d}-OH in which A is an alkyl or alkenyl radical having 8 to 22 carbon atoms, Z is hydrogen or an alkyl radical having 1 to 2 carbon atoms and d is a number from 1 to 40, with the proviso that X and Y are not hydrogen at the same time if R is hydrogen or an alkyl radical with 1 carbon atom, a and/or o is 2 and b and/or p is 1.

2. Use according to Claim 1 for the fixing of fragrances to human skin, hair and/or textiles during washing and/or cleaning, and in bodycare.

3. Use according to Claim 1 or 2, **characterized in that** the fragrances increase the washing and/or cleaning performance of the soil release polymers.

4. Use according to one of Claims 1 to 3, **characterized in that** the soil release polymer is used in amounts by weight, based on the fragrance, in the range from 1:10 to 10:1, in particular from 1:1 to 1:5.

5. Process for the fixing of fragrances to hard and/or soft surfaces, **characterized in that** the surface is treated with the fragrance and a soil release polymer in the presence of water over a period of from 2 minutes to 90 minutes to a temperature below 95°C, with the proviso that the soil release polymer is a polyester and/or copolyester which is chosen from the group comprising
a) copolyesters which comprise dicarboxylic acid units, alkylene glycol units and polyalkylene glycol units
b) polyethylene terephthalate-polyoxyethylene glycol copolymers
c) copolymers of a dibasic carboxylic acid and an alkylene or cycloalkylene polyglycol, and optionally an alkylene or cycloalkylene glycol
d) copolyesters of ethylene glycol, polyethylene glycol, aromatic dicarboxylic acid and sulphonated aromatic dicarboxylic acid
e) methyl- or ethyl-capped polyesters with ethylene terephthalate and/or propylene terephthalate and polyethylene oxide terephthalate units
f) polyesters which, besides oxyethylene groups and terephthalic acid units, also comprise substituted ethylene units and glycerol units
g) polyesters which, besides oxyethylene groups and terephthalic acid units, comprise 1,2-propylene, 1,2-butylene and/or 3-methoxy-1,2-propylene groups and glycerol units and are terminally capped with C₁- to C₄-alkyl groups
h) at least proportionally C₁-C₄-alkyl- or acyl-terminally capped polyesters with polypropylene terephthalate and polyoxyethylene terephthalate units
i) sulphoethyl-terminally capped terephthalate-containing polyesters
j) polyesters prepared by sulphonation of unsaturated end groups with terephthalate, alkylene glycol and/or poly-C₂-C₄-glycol units, and/or
k) polyesters of the general formula (I)
X-(O-(CHR-)ₐ)_{b}[O-OC-Ph-CO-(O-(CHR-)ₒ)ₚ]_{y}O-Y (I)
in which a is a number from 2 to 8, b is a number from 1 to 300, o is a number from 2 to 8, p is a number from 1 to 300 and y is a number from 1 to 500, Ph is an o-, m- or p-phenylene radical which can carry 1 to 4 substituents chosen from alkyl radicals having 1 to 22 carbon atoms, sulphonic acid groups, carboxyl groups and mixtures thereof, R is chosen from hydrogen, an alkyl radical having 1 to 22 carbon atoms and mixtures thereof, and X and Y, independently of one another, are chosen from hydrogen, alkyl- and arylmonocarboxylic acid radicals having 5 to 32 carbon atoms, hydroxymonocarboxylic acid radicals having 2 to 22 carbon atoms and a degree of oligomerization of from 1 to 100, and dicarboxylic monoester radicals whose second carboxylic acid group is esterified by an alcohol A-(OCHZCH₂)_{d}-OH in which A is an alkyl or alkenyl radical having 8 to 22 carbon atoms, Z is hydrogen or an alkyl radical having 1 to 2 carbon atoms and d is a number from 1 to 40, with the proviso that X and Y are not hydrogen at the same time if R is hydrogen or an alkyl radical with 1 carbon atom, a and/or o is 2 and b and/or p is 1.

6. Process according to Claim 5, **characterized in that** the treatment time is in the range from 5 minutes to 60 minutes, in particular from 15 minutes to 45 minutes.

7. Process according to Claim 5 or 6, **characterized in that** the temperature is in the range from 20°C to 60°C, in particular from 20°C to 40°C.

8. Process according to one of Claims 5 to 7, **characterized in that**, during the entire treatment time, the temperature is in the range from 20°C to 40°C.

9. Process according to one of Claims 5 to 8, **characterized in that** the concentration of the soil release polymer is in the range from 0.01 g/l to 0.25 g/l, in particular from 0.05 g/l to 0.15 g/l.

10. Process according to one of Claims 5 to 9, **characterized in that** the concentration of fragrance in the aqueous treatment liquor is in the range from 0.004 g/l to 0.12 g/l, in particular from 0.02 g/l to 0.04 g/l.

## Revendications

1. Utilisation de polymères possédant un pouvoir antisalissure pour la fixation de substances odoriférantes sur des surfaces dures et/ou molles lors du lavage, respectivement du nettoyage, ainsi que lors des soins corporels, **caractérisée en ce qu'**il s'agit, en ce qui concerne les polymères possédant un pouvoir antisalissure, de polyesters et/ou de copolyesters qui sont choisis parmi le groupe comprenant
a) des copolyesters qui contiennent des unités d'acides dicarboxyliques, des unités d'alkylèneglycols et des unités de polyalkylèneglycols
b) des copolymères de polyéthylènetéréphtalate-polyoxy-éthylèneglycol
c) un copolymère d'un acide carboxylique dibasique et d'un alkylène- ou d'un cycloalkylène-polyglycol, et éventuellement d'un alkylène- ou d'un cycloalkylène-glycol
d) un copolyester d'éthylèneglycol, de polyéthylèneglycol, d'acide dicarboxylique aromatique et d'acide dicarboxylique aromatique sulfoné
e) un polyester dont les extrémités terminales sont constituées par un groupe méthyle ou un groupe éthyle, comprenant des unités d'éthylène- et/ou de propylène-téréphtalate et des unités d'oxyde de polyéthylène-téréphtalate
f) un polyester qui contient, outre des groupes d'oxyéthylène et des unités d'acide téréphtalique, également des unités d'éthylène substituées, ainsi que des unités de glycérol
g) des polyesters qui contiennent, outre des groupes d'oxyéthylène et des unités d'acide téréphtalique, des groupes de 1,2-propylène, de 1,2-butylène et/ou de 3-méthoxy-1,2-propylène, ainsi que des unités de glycérol, et dont les extrémités terminales sont constituées par des groupes alkyle en C₁-C₄
h) des polyesters dont les extrémités terminales sont constituées, au moins en partie, par des radicaux alkyle en C₁-C₄ ou par des radicaux acyle, comprenant des unités de polypropylène-téréphtalate et de polyoxyéthylène-téréphtalate
i) des polyesters contenant des groupes téréphtalate, dont les extrémités terminales sont constituées par des groupes sulfoéthyle
j) des polyesters, préparés par sulfonation de groupes terminaux insaturés comprenant des unités de téréphtalate, d'alkylèneglycols et de polyglycols en C₂-C₄ et/ou
k) des polyesters répondant à la formule générale (I)
X-(O-(CHR-)ₐ)_{b}[O-OC-Ph-CO-(O-(CHR-)ₒ)ₚ)_{y}]O-Y (I)
dans laquelle a représente un nombre de 2 à 8, b représente un nombre de 1 à 300, o représente un nombre de 2 à 8, p représente un nombre de 1 à 300 et y représente un nombre de 1 à 500, Ph représente un radical o-phénylène, m-phénylène ou p-phénylène qui peut porter de 1 à 4 substituants choisis parmi le groupe comprenant des radicaux alkyle contenant de 1 à 22 atomes de carbone, des groupes d'acides sulfoniques, des groupes carboxyle et leurs mélanges, R est choisi parmi le groupe comprenant un atome d'hydrogène, un radical alkyle contenant de 1 à 22 atomes de carbone et leurs mélanges, et X et Y sont choisis, indépendamment l'un de l'autre, parmi le groupe comprenant un atome d'hydrogène, des radicaux d'acides alkyl- et aryl-monocarboxyliques contenant de 5 à 32 atomes de carbone, des radicaux d'acides hydroxy-monocarboxyliques contenant de 2 à 22 atomes de carbone et possédant un degré d'oligomérisation de 1 à 100, ainsi que des radicaux de demi-esters d'acides dicarboxyliques dont le deuxième groupe d'acide carboxylique a été estérifié avec un alcool A-(OCHZCH₂)_{d}-OH dans lequel A représente un radical alkyle ou un radical alcényle contenant de 8 à 22 atomes de carbone, Z représente un atome d'hydrogène ou un radical alkyle contenant de 1 à 2 atomes de carbone et d représente un nombre de 1 à 40, avec cette mesure que X et Y ne représentent pas simultanément un atome d'hydrogène lorsque R représente un atome d'hydrogène ou un radical alkyle contenant 1 atome de carbone, a et/ou o représentent 2 et b et/ou p représentent 1.

2. Utilisation selon la revendication 1, pour la fixation de substances odoriférantes sur la peau humaine, sur les cheveux et/ou sur des textiles lors du lavage, respectivement du nettoyage, ainsi que lors des soins corporels.

3. Utilisation selon la revendication 1 ou 2, **caractérisée en ce que**, via les substances odoriférantes, on augmente le rendement de lavage et/ou de nettoyage des polymères possédant un pouvoir antisalissure.

4. Utilisation selon l'une quelconque des revendications 1 à 3, **caractérisée en ce qu'**on met en oeuvre le polymère possédant un pouvoir antisalissure dans des quantités pondérales, rapportées à la substance odoriférante, dans la plage de 1 : 10 à 10 : 1, en particulier de 1 : 1 à 1 : 5.

5. Procédé pour la fixation de substances odoriférantes sur des surfaces dures et/ou molles, **caractérisé en ce qu'**on traite la surface avec la substance odoriférante et un polymère possédant un pouvoir antisalissure en présence d'eau pendant un laps de temps de 2 minutes à 90 minutes à une température inférieure à 95 °C, avec cette mesure qu'en ce qui concerne le polymère possédant un pouvoir antisalissure, il s'agit d'un polyester et/ou d'un copolyester qui est choisi parmi le groupe comprenant
a) des copolyesters qui contiennent des unités d'acides dicarboxyliques, des unités d'alkylèneglycols et des unités de polyalkylèneglycols
b) des copolymères de polyéthylènetéréphtalate-polyoxy-éthylèneglycol
c) un copolymère d'un acide carboxylique dibasique et d'un alkylène- ou d'un cycloalkylène-polyglycol, et éventuellement d'un alkylène- ou d'un cycloalkylène-glycol
d) un copolyester d'éthylèneglycol, de polyéthylèneglycol, d'acide dicarboxylique aromatique et d'acide dicarboxylique aromatique sulfoné
e) un polyester dont les extrémités terminales sont constituées par un groupe méthyle ou un groupe éthyle, comprenant des unités d'éthylène- et/ou de propylène-téréphtalate et des unités d'oxyde de polyéthylène-téréphtalate
f) un polyester qui contient, outre des groupes d'oxyéthylène et des unités d'acide téréphtalique, également des unités d'éthylène substituées, ainsi que des unités de glycérol
g) des polyesters qui contiennent, outre des groupes d'oxyéthylène et des unités d'acide téréphtalique, des groupes de 1,2-propylène, de 1,2-butylène et/ou de 3-méthoxy-1,2-propylène, ainsi que des unités de glycérol, et dont les extrémités terminales sont constituées par des groupes alkyle en C₁-C₄
h) des polyesters dont les extrémités terminales sont constituées, au moins en partie, par des radicaux alkyle en C₁-C₄ ou par des radicaux acyle, comprenant des unités de polypropylène-téréphtalate et de polyoxyéthylène-téréphtalate
i) des polyesters contenant des groupes téréphtalate, dont les extrémités terminales sont constituées par des groupes sulfoéthyle
j) des polyesters, préparés par sulfonation de groupes terminaux insaturés comprenant des unités de téréphtalate, d'alkylèneglycols et de polyglycols en C₂-C₄ et/ou
k) des polyesters répondant à la formule générale (I)
X-(O-(CHR-)ₐ)_{b}[O-OC-Ph-CO-(O-(CHR-)ₒ)ₚ)_{y}]O-Y (I)
dans laquelle a représente un nombre de 2 à 8, b représente un nombre de 1 à 300, o représente un nombre de 2 à 8, p représente un nombre de 1 à 300 et y représente un nombre de 1 à 500, Ph représente un radical o-phénylène, m-phénylène ou p-phénylène qui peut porter de 1 à 4 substituants choisis parmi le groupe comprenant des radicaux alkyle contenant de 1 à 22 atomes de carbone, des groupes d'acides sulfoniques, des groupes carboxyle et leurs mélanges, R est choisi parmi le groupe comprenant un atome d'hydrogène, un radical alkyle contenant de 1 à 22 atomes de carbone et leurs mélanges, et X et Y sont choisis, indépendamment l'un de l'autre, parmi le groupe comprenant un atome d'hydrogène, des radicaux d'acides alkyl- et aryl-monocarboxyliques contenant de 5 à 32 atomes de carbone, des radicaux d'acides hydroxy-monocarboxyliques contenant de 2 à 22 atomes de carbone et possédant un degré d'oligomérisation de 1 à 100, ainsi que des radicaux de demi-esters d'acides dicarboxyliques dont le deuxième groupe d'acide carboxylique a été estérifié avec un alcool A-(OCHZCH₂)_{d}-OH dans lequel A représente un radical alkyle ou un radical alcényle contenant de 8 à 22 atomes de carbone, Z représente un atome d'hydrogène ou un radical alkyle contenant de 1 à 2 atomes de carbone et d représente un nombre de 1 à 40, avec cette mesure que X et Y ne représentent pas simultanément un atome d'hydrogène lorsque R représente un atome d'hydrogène ou un radical alkyle contenant 1 atome de carbone, a et/ou o représentent 2 et b et/ou p représentent 1.

6. Procédé selon la revendication 5, **caractérisé en ce que** le temps de traitement se situe dans la plage de 5 minutes à 60 minutes, en particulier de 15 minutes à 45 minutes.

7. Procédé selon la revendication 5 ou 6, **caractérisé en ce que** la température se situe dans la plage de 20 °C à 60 °C, en particulier de 20 °C à 40 °C.

8. Procédé selon l'une quelconque des revendications 5 à 7, **caractérisé en ce que**, pendant tout le temps de traitement, la température se situe dans la plage de 20 °C à 40 °C.

9. Procédé selon l'une quelconque des revendications 5 à 8, **caractérisé en ce que**, la concentration du polymère possédant un pouvoir antisalissure se situe dans la plage de 0,01 g/litre à 0,25 g/litre, en particulier de 0,05 g/litre à 0,15 g/litre.

10. Procédé selon l'une quelconque des revendications 5 à 9, **caractérisé en ce que**, la concentration de substance odoriférante dans le bain de traitement aqueux se situe dans la plage de 0,004 g/litre à 0,12 g/litre, en particulier de 0,02 g/litre à 0,04 g/litre.
